# EUROPEAN PATENT SPECIFICATION

(11) **EP 3 580 229 B1**
(45) Date of publication and mention of the grant of the patent: **29.11.2023**
(21) Application number: 18705859.9
(22) Date of filing: 08.02.2018
(51) Int. Cl.: C07K 14/195, C12N 7/00

(54) **REBOOTING OF SYNTHETIC BACTERIOPHAGE GENOME IN L-FORM BACTERIA**
NEUSTART VON BAKTERIOPHAGE GENOMEN IN L-FORMIGE BAKTERIEN
RÉACTIVATION DU GÉNOME DE BACTERIOPHAGE SYNTHÉTIQUE DANS LES BACTÉRIES EN FORME L

(30) Priority: 10.02.2017 EP 17155693
(43) Date of publication of application: 18.12.2019
(73) Proprietor: ETH Zurich, 8092 Zurich (CH)
(72) Inventor: LOESSNER, Martin Johannes, 8123 Ebmatingen (CH); KILCHER, Samuel, 8803 Rüschlikon (CH); STUDER, Patrick, 8049 Zürich (CH)
(74) Representative: Nederlandsch Octrooibureau
(86) International application number: PCT/EP2018/053202
(87) International publication number: WO 2018/146206

(56) References cited:
- SIMONE DELL'ERA ET AL: "Listeria monocytogenes l-forms respond to cell wall deficiency by modifying gene expression and the mode of division", MOLECULAR MICROBIOLOGY., vol. 73, no. 2, 1 July 2009 (2009-07-01), pages 306-322, XP055356980, GB ISSN: 0950-382X, DOI: 10.1111/j.1365-2958.2009.06774.x
- PATRICK STUDER ET AL: "Proliferation of Listeria monocytogenes L-form cells by formation of internal and external vesicles", NATURE COMMUNICATIONS, vol. 7, 23 November 2016 (2016-11-23), page 13631, XP055356991, United Kingdom ISSN: 2041-1723, DOI: 10.1038/ncomms13631
- T B White ET AL: "Transformation of a Bacillus subtilis L-form with bacteriophage deoxyribonucleic acid", Journal of Bacteriology, 1 February 1981 (1981-02-01), pages 878-883, XP055356785, UNITED STATES Retrieved from the Internet: URL:https://www.ncbi.nlm.nih.gov/pmc/artic les/PMC217193/pdf/jbacter00273-0214.pdf
- J. GUMPERT ET AL: "Phage adsorption and productive lysis in stable protoplast type L-forms ofBacillus subtilis andStreptomyces hygroscopicus", JOURNAL OF BASIC MICROBIOLOGY, vol. 26, no. 1, 1 January 1986 (1986-01-01), pages 15-25, XP055356495, BERLIN, DE ISSN: 0233-111X, DOI: 10.1002/jobm.3620260106
- A HIBMA: "Infection and removal of L-forms of Listeria monocytogenes with bred bacteriophage", INTERNATIONAL JOURNAL OF FOOD MICROBIOLOGY, vol. 34, no. 3, 3 March 1997 (1997-03-03), pages 197-207, XP055356511, NL ISSN: 0168-1605, DOI: 10.1016/S0168-1605(96)01190-7

## Description

### Field

The present disclosure relates to a method and means for producing or propagating engineered bacteriophages.

### Background

Bacteriophages (phages) are viruses that exclusively infect bacteria and constitute their natural enemies. Most phages infect a specific subset of strains of a given bacterial species without targeting other, closely related bacteria. Due to their extraordinary specificity and bacteriolytic potential, phages are employed for a variety of biomedical and biotechnological applications. On the one hand, they are used as diagnostic tools for rapid and sensitive detection of live bacterial pathogens. On the other hand, virulent phages are applied for the biocontrol of specific species, effectively removing potential pathogens from industrial production chains, and food products. Also, due to the rise of antibiotic resistance with an estimated 48'000 deaths per year in the EU and USA (WHO and CDC), the application of phage as an alternative antimicrobial is a re-emerging field of interest and phage therapy approaches show promising results. Considering the low discovery rates of traditional antibiotics combined with the increasing public health threat resistant bacteria present, it is reasonable to assume that the renaissance of phage therapy will continue. Despite their high genus- and species-specificity, self-replicating nature and low production cost, phages face a number of challenges that still limit their use in a biomedical setting: Due to restricted host-ranges of individual phages, phage cocktails need to be designed to cover all medically relevant strains of a pathogenic species. Obtaining regulatory approval for phage-products in general and phage cocktails in particular is challenging and the regulatory framework unclear. In addition, lysogenic (temperate) phages can integrate into the host genome without inducing cell lysis and may even contribute to the spread of antibiotic resistance by transduction or increase bacterial virulence by lysogenic conversion, effectively excluding their use as biocontrol agents. Also, target cells can encode a plethora of phage resistance-mechanisms including receptor diversification, biofilm-formation, and CRISPR interference to mention a few. By modifying the genomes of phages, many of these limitations can be overcome and additional beneficial properties can be included into a phage genome.

Unfortunately, genome engineering of virulent phages is a difficult and work-intensive process at best. In most cases, phage genomes are modified during infection by homologous recombination with a plasmid carrying homologous sequences and the desired genetic alteration. Because phage replication is fast and recombination rates often very low (10⁻⁴ to 10⁻¹⁰), screening for recombinant phage is labor-intensive and requires the incorporation of reporter genes. Recently, researchers have presented an elegant synthetic approach to modify phage genomes: Yeast artificial chromosomes (YAC) and overlapping phage genome fragments were assembled *in vivo* in yeast cells to produce a complete recombinant genome captured in a YAC. Subsequently, YAC-phage DNA was transformed into *E. coli* to reboot these phages. So far, the approach is limited to viruses of the T7-family which infect Gram-negative cells and feature host-independent replication. Whether similar approaches are adaptable to different phage-families and/or to phages infecting Gram-positive cells is currently unknown.

Accordingly, there is a need for simple and efficient methods to produce complete recombinant phage genomes that are applicable also for phages with Gram-positive hosts.

### Detailed Description

Based on the above described background, it is the objective of the present disclosure to provide simple and efficient methods and means for propagating engineered bacteriophages, particularly including bacteriophages that infect Gram-positive host cells.

This objective is attained by a method according to claim 1 or claim 10 and a cell wall-deficient bacterium according to claim 11.

According thereto, a first aspect of the disclosure relates to a method for producing or propagating an engineered bacteriophage, comprising the steps of:
- providing a functional synthetic genome of an engineered bacteriophage being able to infect a target bacterium,
- providing a recipient bacterium,
- transforming said recipient bacterium with said functional synthetic genome in a transformation step, yielding a transformed recipient bacterium,
- incubating said transformed recipient bacterium in a first incubation step, wherein said engineered bacteriophage is propagated within said transformed recipient bacterium, and
- further incubating said transformed recipient bacterium or said propagated engineered bacteriophage released from said transformed recipient bacterium with a target bacterium in a second incubation step, wherein said propagated engineered bacteriophage infects said target bacterium and is further propagated within said target bacterium,
wherein said recipient bacterium is a cell wall-deficient bacterium.

The term "cell wall-deficient" bacterium in the context of the present specification refers to a cell wall-deficient variant of a otherwise walled bacterium that proliferate actively in the cell wall-deficient state in osmotically stabilized media. They lack the multi-layered peptidoglycan envelope which usually restricts transformation of, for example, Gram-positive bacteria with large DNA molecules. Cell wall-deficient bacteria are also known as L-form bacteria, L-phase bacteria or L-phase variants.

Accordingly, such cell wall-deficient bacterium is particularly a metabolic active cell wall-deficient bacterium and/or a cell wall-deficient bacterium that is able to actively proliferate. Particularly, such cell wall-deficient bacterium may be transiently or permanently cell wall-deficient.

The term "synthetic genome" in the context of the present specification particularly refers to an artificial or non naturally occurring genome. Likewise, the term "engineered bacteriophage" in the context of the present specification particularly refers to an artificial or non naturally occurring bacteriophage, particularly characterized by a synthetic genome.

Particularly, the functional genome is transformed in form of a "naked" nucleic acid or "naked" nucleic acids, e.g. without a protein capsid or envelope.

Alternatively, the propagated bacteriophage is incubated with the target bacterium in the second incubation step, wherein the propagated bacteriophage is released from the transformed recipient bacterium, particularly by lysing the transformed recipient bacterium, e.g. by an osmotic shock.

The method of the disclosure is a novel approach for the production, propagation, reactivation or engineering of recombinant bacteriophages that is much faster and more reliable when compared to the current state-of-the-art. This approach does not require screening for correct recombinants or the incorporation of a detectable reporter-gene. In addition, it is applicable to a very broad range of phages infecting Gram-positive organisms. This method is a big step forward towards the generation of tailored bacteriophages with desired biomedical and biotechnological properties. Advantageously, the method of the disclosure circumvents limitations of known methods and is thus broadly applicable to phages infecting Gram-positive organisms.

Particularly, the transformation step and/or the first incubation step is conducted in an osmoprotective medium.

The term "osmoprotective medium" in the context of the present specification particularly refers to a medium, which enables the survival and/or growth of the cell wall-deficient bacterium and further comprises a non-toxic, water soluble, osmotic active compound, particularly in a concentration at which the osmotic pressure between the cell wall-deficient bacterium and the medium is below a threshold above which rupture of the cell wall-deficient bacterium occurs. Non-limiting examples for such compounds include non-toxic organic acid or salts thereof, such as succinate, carbohydrates, or non-toxic salts, such as ammonium sulfate or sodium chloride.

Particularly, the second incubation step is conducted in absence of an osmoprotective medium.

The transformation step may be conducted in a liquid medium. The first incubation step may be conducted in a liquid medium. The second incubation step may be conducted in a liquid medium.

The osmoprotective medium may comprise succinate, particularly in a concentration ranging from 0.075 mol*l⁻¹ to 0.5 mol*l⁻¹. The osmoprotective medium may comprise a monosaccharide, a disaccharide or a trisaccharide, particularly glucose, or sucrose. The osmoprotective medium may comprise glycerin. The osmoprotective medium may comprise sucrose in a concentration in the range of 0.25 mol*l⁻¹ to 0.75 mol*l⁻¹, particularly 0.5 mol*l⁻¹ .

In certain embodiments, the recipient bacterium is a cell wall-deficient variant of or derived from a Gram-positive bacterium.

The term "derived from" in the context of the present specification particularly refers to a process by which a respective bacterium, e.g. a parental cell walled Gram-positive bacterium, is transformed into a cell wall-deficient bacterium by means of, for example, culture conditions such as presence of a cell wall synthesis interfering antibiotic and/or in presence of an osmoprotective medium.

In certain embodiments, the recipient bacterium is a cell wall-deficient bacterium of the genus *Listeria.* In certain embodiments, the recipient bacterium is a cell wall-deficient variant of or derived from *Listeria monocytogenes.* The recipient bacterium may be a cell wall-deficient variant of or derived from *Listeria monocytogenes* EGD-e. The recipient bacterium may be a cell wall-deficient variant of or derived from *Listeria innocua.* The recipient bacterium may be a cell wall-deficient variant of or derived from *Listeria innocua* 2021, particularly from *Listeria innocua* strain SLCC 5639 (Special Listeria Culture Collection, Univ. of Wurzburg, Germany).

In certain embodiments, the target bacterium is a Gram-positive bacterium. In certain embodiments, the target bacterium is selected from the genus *Listeria, Bacillus Enterococcus, Streptococcus, Clostridium* or *Staphylococcus.* In certain embodiments, the target bacterium is selected from *Listeria monocytogenes, Listeria ivanovii, Listeria innocua, Bacillus subtilis, Bacillus cereus, Bacillus thuringiensis* or *Staphylococcos aureus.*

In certain embodiments, the recipient bacterium and the target bacterium belong to or are derived from different species. In certain embodiments, the recipient bacterium and the target bacterium belong to or are derived from members of different genera.

The recipient bacterium may be derived from a member of the genus Listeria, and the target bacterium may belong to the genus selected from *Bacillus Enterococcus, Streptococcus, Clostridium* or *Staphylococcus.*

The functional genome may be a synthetic or artificial bacteriophage genome, particularly of an engineered bacteriophage.

The term "synthetic or artificial bacteriophage genome" in the context of the present specification particularly refers to an artificial non-naturally occurring nucleic acid construct.

Such synthetic or artificial bacteriophage genome may originate from a naturally occurring bacteriophage genome in which one or more foreign genetic elements such as genes, regulatory elements (e.g. promoters), operons, or open reading frames have be incorporated, and/or naturally occurring genetic elements have been replaced, modified and/or deleted. Such synthetic bacteriophage genome may also be a mosaic of a plurality of genetic elements originating from a plurality of different organisms.

In certain embodiments, the functional genome, particularly the functional synthetic or artificial genome, is provided by *in vitro* or *in vivo* assembly of fragments thereof. Particularly, fragments of the functional genome may be provided by *de novo* synthesis, cloning or amplification, wherein the provided fragments then may be assembled into the functional genome by methods known in the art. A non-limiting example for *in vitro* assembly is the Gibson assembly, wherein the aforementioned fragments share overlapping sequences upon which they are assembled. A non-limiting example for *in vivo* assembly is the yeast assembly, wherein a yeast cell is transformed with the aforementioned fragments, also comprising overlapping sequences, and the fragments are assembled within the yeast cell.

In certain embodiments, the functional genome, particularly the function synthetic or artificial genome, is provided by *de novo* synthesis.

The synthetic or artificial bacteriophage genome may originate from a temperate bacteriophage, wherein the synthetic or artificial bacteriophage genome lacks the gene for the repressor of the lytic cycle or the lysogeny control region.

The functional genome may be a linear or circular nucleic acid molecule. The functional genome may be a single-stranded or double-stranded RNA or DNA molecule.

In certain embodiments, the functional genome has a length of at least 10.000 base pairs In certain embodiments, the functional genome has a length of at least 30.000 base pairs. In certain embodiments, the functional genome has a length of at least 40.000 base pairs. The functional genome may have a length of at least 120.000 base pairs. The functional genome may have a length in range of 10,000 base pairs to 160,000 base pairs. The functional genome may have a length in range of 35,000 base pairs to 160,000 base pairs. The functional genome may have a length in range of 40,000 base pairs to 130,000 base pairs.

The functional genome may originate from a Siphovirus, particularly Siphovirus TP21-L, Siphovirus 2638A, Siphovirus P35, Siphovirus B025, Siphovirus B035, Siphovirus B056, Siphovirus PSA, or Siphovirus P70, or a Myovirus, particularly Myovirus A511, Myovirus P100, Myovirus Bastille, or phage K, or a podovirus.

In certain embodiments, the transformation step is conducted in presence of a polyethylene glycol. In certain embodiments, the polyethylene glycol has a mean molecular weight in the range of 1,000 g*mol⁻¹ to 30,000 g*mol⁻¹. In certain embodiments, the polyethylene glycol has a mean molecular weight in the range of 7,000 g*mol⁻¹ to 20,000 g*mol⁻¹. In certain embodiments, the polyethylene glycol is a PEG-8000. The transformation step may be conducted in presence of a polyethylene glycol in a concentration ranging from to approx. 6 % (w/v) to approx. 36 % (w/v). The transformation step may be conducted in presence of a polyethylene glycol in a concentration of approx. 24 % (w/v).

The term "mean molecular weight" with regard to polyethylene glycol particularly refers to the arithmetic mean or to the median of the molecular weight distribution of the respective polyethylene glycol. Such mean molecular weight may be determined by methods known to the skilled person such as, for example, by static or dynamic light scattering (SLS, DLS), size-exclusion chromatography, or gel electrophoresis.

In certain embodiments, the first incubation step is conducted over a period in the range of 4 h to 96 h. In certain embodiments, the first incubation step is conducted over a period in the range of 24 h to 32 h.

The first incubation step may be conducted at a temperature in the range of 15 °C to 37 °C, particularly in the range of 20 °C to 32 °C. The second incubation step may be conducted at a temperature in the range of 15 °C to 37 °C, particularly in the range of 20 °C to 32 °C. The transformation step may be conducted at a temperature in the range of 15 °C to 37 °C, particularly in the range of 20 °C to 37 °C.

In certain embodiments, the recipient bacterium is provided by incubating a cell walled precursor bacterium in presence of a cell wall synthesis interfering antibiotic yielding the recipient bacterium. Particularly, the precursor bacterium is incubated in presence of a cell wall synthesis interfering antibiotic over a period being larger than the doubling time of the precursor bacterium at the present conditions. The precursor bacterium may be incubated in presence of a cell wall synthesis interfering antibiotic over a period in the range of 2 days to 5 days, particularly in the range of 3 days to 4 days.

In certain embodiments, the cell wall synthesis interfering antibiotic is may be selected from beta lactam antibiotics, glycopeptide antibiotic, fosfomycin or cycloserine.

The beta lactam antibiotic may be selected from cephalosporins carbacephems, monobactams, or penicillins.

The cell wall synthesis interfering antibiotic may be penicillin G.

Alternatively, the recipient bacterium may be provided by inhibiting proteins relevant for the cell wall synthesis on the protein level or the genomic level within the cell walled precursor bacterium, or by incubating the cell walled precursor bacterium in presence of cell wall degrading or lysing enzyme such as lysozyme and in presence of an osmoprotective medium.

According to another aspect of the disclosure, a method for manufacturing a cell wall-deficient bacterium of the genus *Listeria* is provided. The method comprises the steps of:
- providing a bacterium of the genus *Listeria,* particularly *Listeria monocytogenes* and more particularly *Listeria monocytogenes* EGD-e, characterized by the genotype [Δ*lmo0584 Δlmo1653-54 Δlm01861*] or by a genome not comprising functional homologues of *lmo0584, lmo1653-54* and *lmo01861;* or
- providing a bacterium of the species *Listeria innocua,* particularly a bacterium of the strain 2021 of *Listeria innocua;* and
cultivating said bacterium in presence of a cell wall synthesis interfering antibiotic selected from the group comprising beta lactam antibiotics, glycopeptide antibiotics, cycloserine, or fosfomycin, particularly penicillin G and optionally in presence of an osmoprotective medium.

The term *"lmo0584"* refers to a gene in *Listeria monocytogenes* EGD-e (Gene ID: 984661, NCBI Gene database).

The term *"lmo1653-54* " refers to two genes in *Listeria monocytogenes* EGD-e (Gene ID: 985674 and Gene ID: 985673, NCBI Gene database).

The term *"lmo01861"* refers to a gene in *Listeria monocytogenes* EGD-e (Gene ID: 985831, NCBI Gene database).

The term "functional homologue" refers in the context of the present specification to gene that has an identical function but differs in the nucleic acid sequence.

In certain embodiments, the bacterium of the genus *Listeria* is provided by inactivation the genes *lmo0584, lmo1653-54* and *lmo01861* or homologues thereof. The inactivation of the genes *lmo0584, lmo1653-54* and *lmo01861* or homologues thereof may be performed by deletion of the genes or homologues thereof, base substitutions, deletions, insertions, or sequences inversion within the genes or homologues thereof.

The cell wall synthesis interfering antibiotic may be selected from beta lactam antibiotics, particularly cephalosporins, carbacephems, monobactams, or penicillins, glycopeptide antibiotics, fosfomycin or cycloserine.

In certain embodiments, the bacterium of the genus *Listeria* is *Listeria monocytogenes.* In certain embodiments, the bacterium of the genus *Listeria* is *Listeria monocytogenes* strain EGD-e.

The bacterium may be cultivated at a temperature in the range of 20 °C to 32 °C.

Alternatively, the cell wall-deficient bacterium may be manufactured by inhibiting proteins relevant for the cell wall synthesis on the protein level or the genomic level, or by incubating a walled bacterium in presence of cell wall degrading or lysing enzyme such as lysozyme and in presence of an osmoprotective medium.

According to yet another aspect of the disclosure, a cell wall-deficient bacterium of the genus *Listeria* is provided, wherein the bacterium is characterized by the genotype [Δ*lmo0584 Δlmo1653-54 Δlm01861*] or by a genome not comprising functional homologues of *lmo0584, lmo1653-54* and *lmo01861.*

In certain embodiments, the cell wall-deficient bacterium is *Listeria monocytogenes.* In certain embodiments, the cell wall-deficient bacterium is *Listeria monocytogenes* strain EGD-e.

The cell wall-deficient bacterium may be obtainable or obtained by a method according to the above aspect of the disclosure.

Wherever alternatives for single separable features are laid out herein as "embodiments", it is to be understood that such alternatives may be combined freely to form discrete embodiments of the disclosure disclosed herein.

The disclosure is further illustrated by the following examples and figures, from which further embodiments and advantages can be drawn. These examples are meant to illustrate the disclosure but not to limit its scope.

### Description of the figures

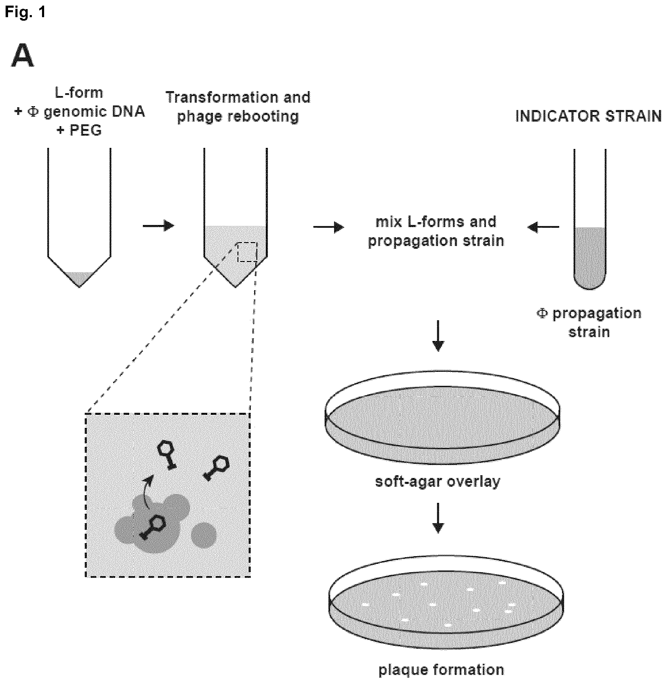

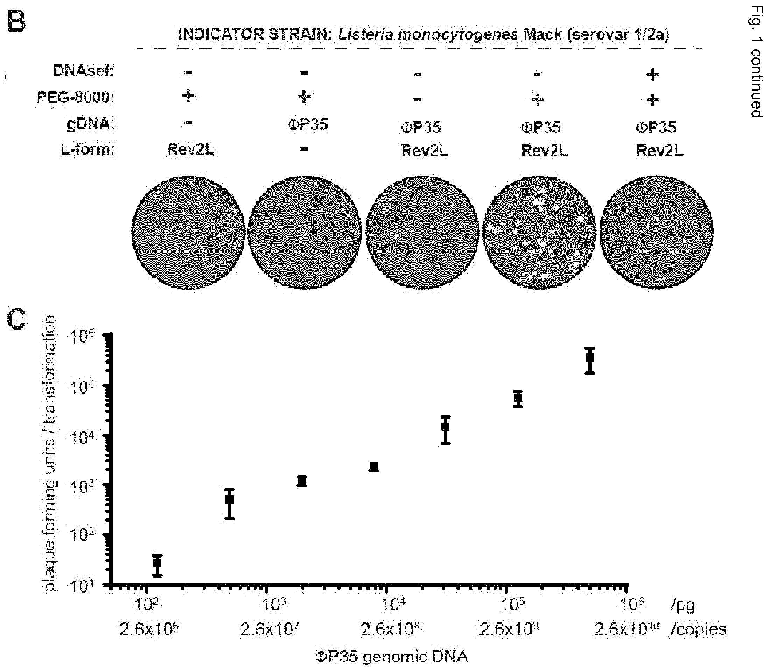

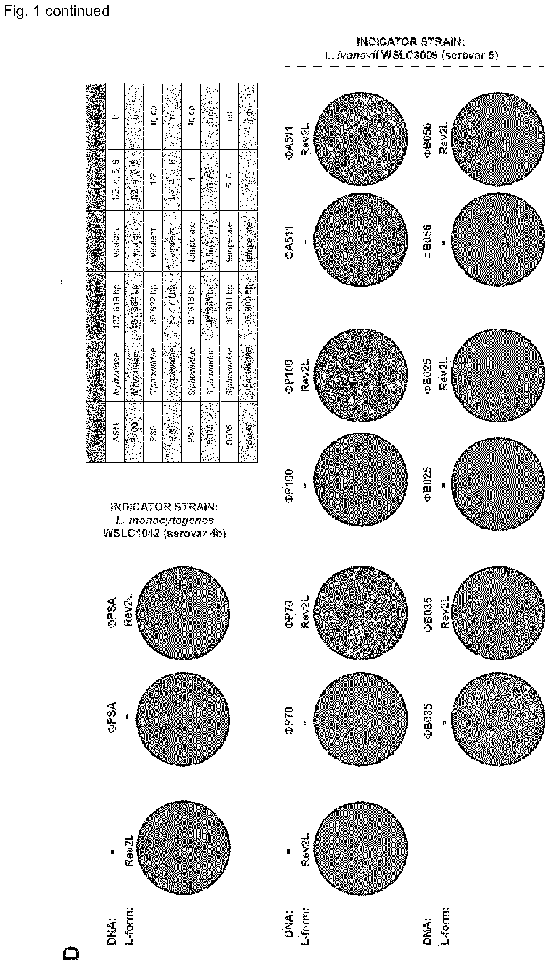

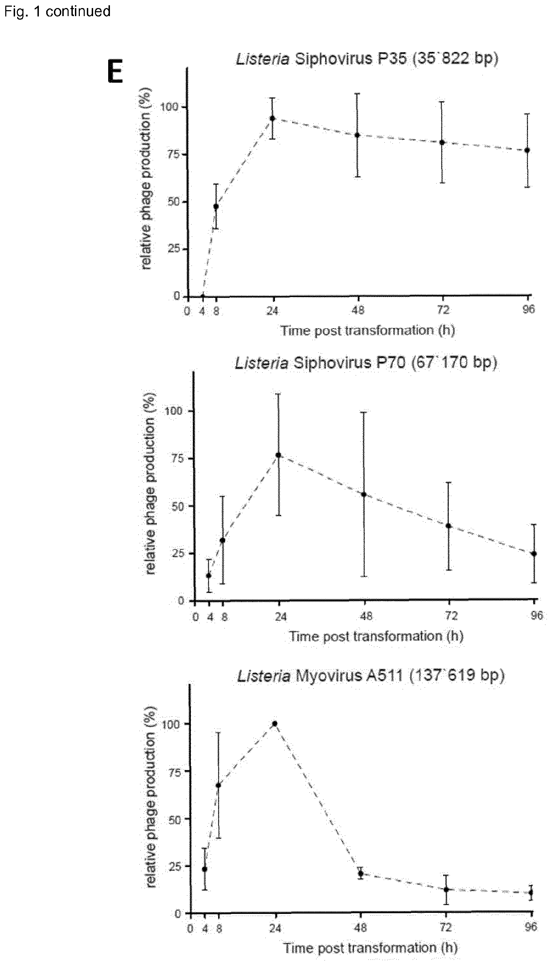

- Fig. 1: shows the rebooting of *Listeria* bacteriophage genomes in L-form strain Rev2L. (A-B) The ability to reboot *Listeria* phages in L-form strain Rev2L was assessed as depicted in (A) using genomic DNA of *Listeria* phage P35. L-form transformation reactions were prepared as indicated in (B), incubated at 32°C and tested for plaque formation on the indicator strain at 24 h post transformation. DNAsel indicates a 30 min pre-digestion of P35 gDNA using DNAsel. (C) Efficiency of transformation and rebooting was determined using a dilution series of P35 gDNA. (D) A set of seven additional *Listeria* phages was rebooted in Rev2L using 500 - 1000 ng gDNA and detected using *Listeria monocytogenes* or *Listeria ivanovii* as indicator strains. Phage properties are listed in tabular form. (E) Bacteriophage production kinetics in L-forms were assessed for three phages over a period of 96 h. Data are mean ± SD (n=3). Φ = phage, PEG = polyethylene glycol, gDNA = genomic DNA, tr = terminal redundancy, cp = circular permutation, cos = cohesive end site, nd = not determined.

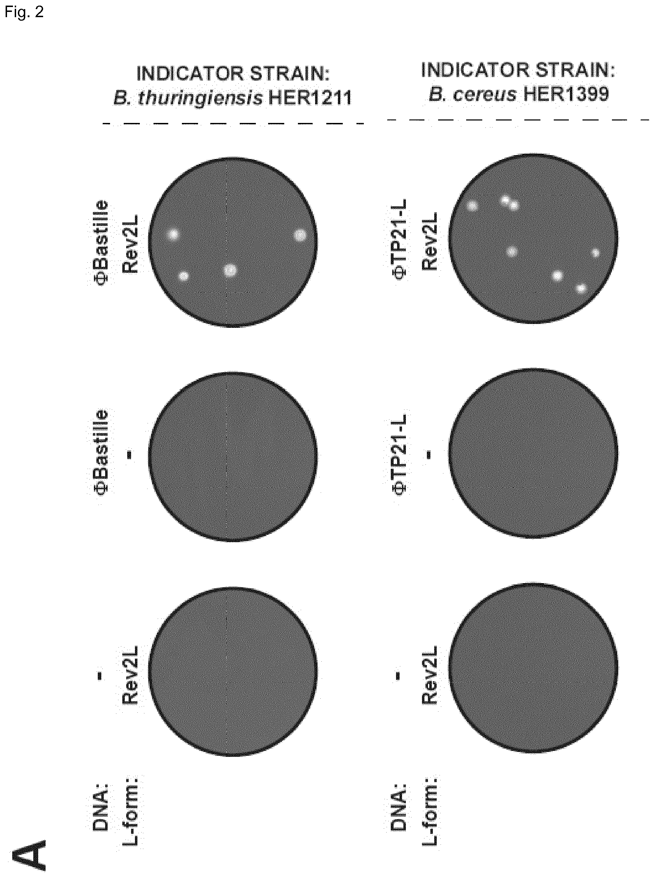

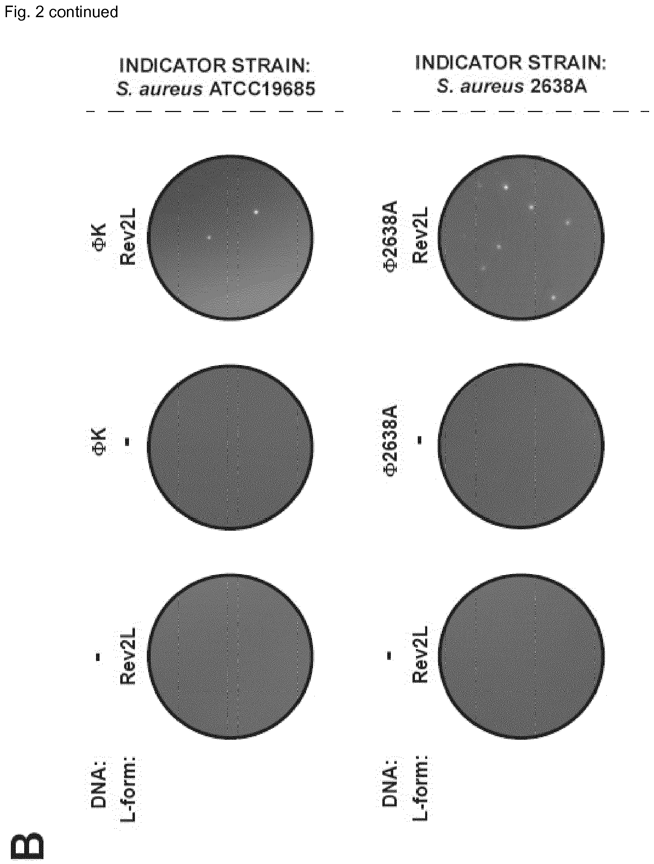

- Fig. 2: shows the rebooting of (A) *Bacillus* and (B) *Staphylococcus* phage genomes in *Listeria* L-forms. Rev2L cells were transformed with 1 - 5 µg of phage gDNA, incubated for 24 h at 32 °C and assayed for phage production on their respective indicator strains. Transformation reactions lacking either the gDNA or the L-form cells served as controls. Φ = phage.

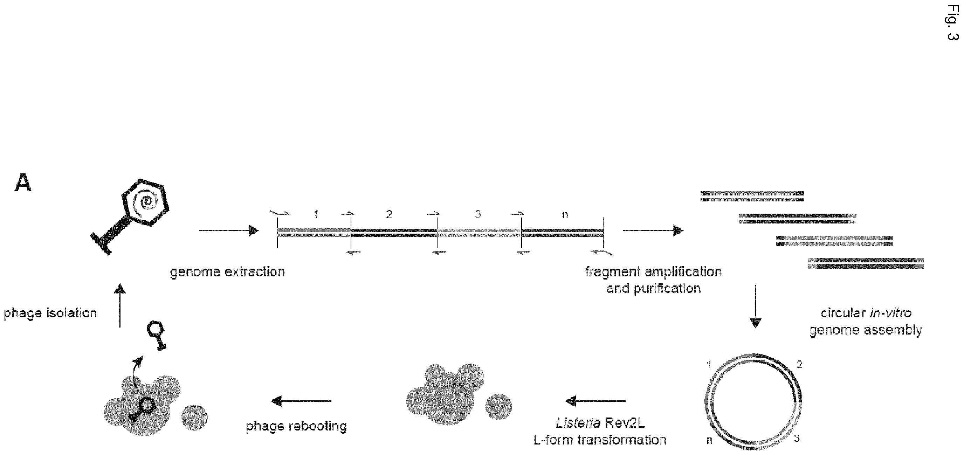

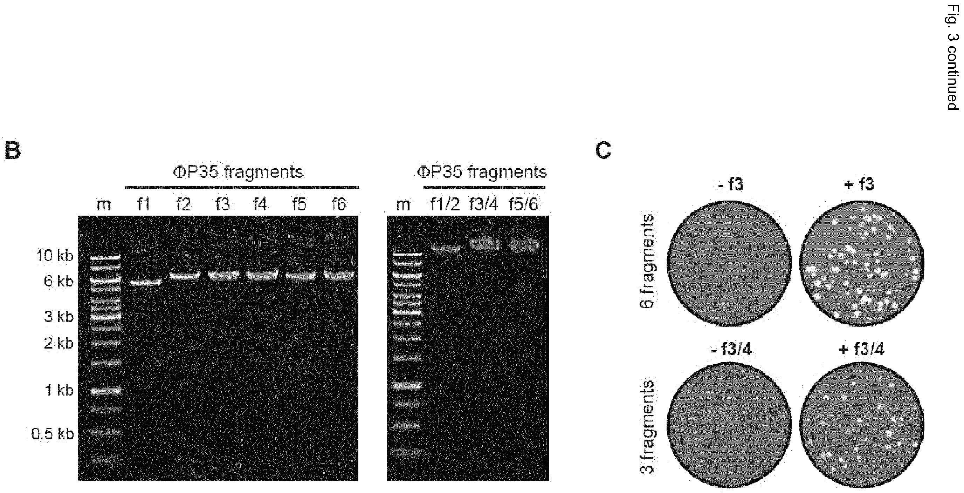

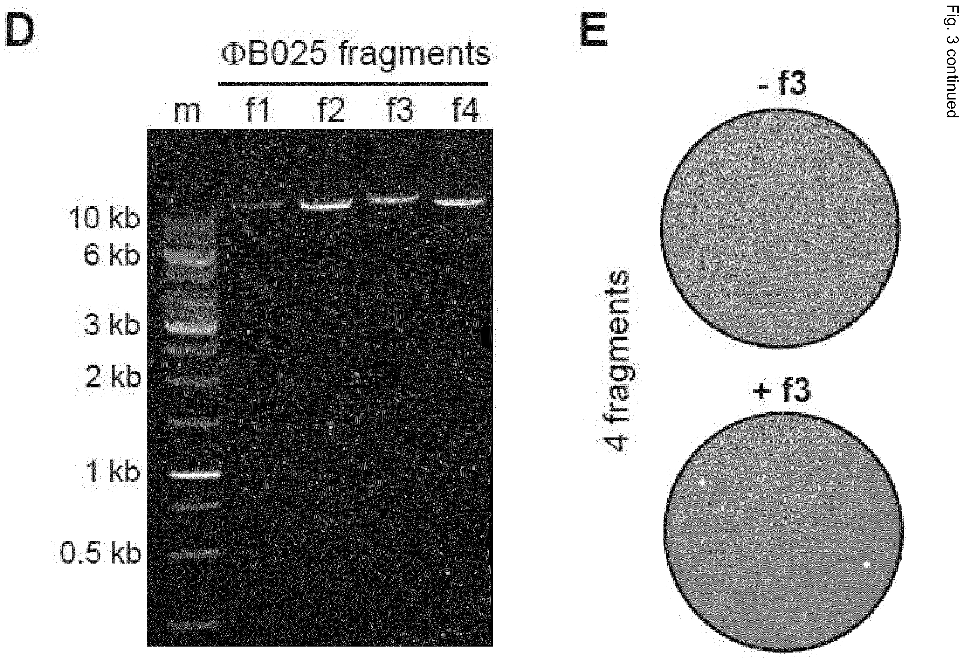

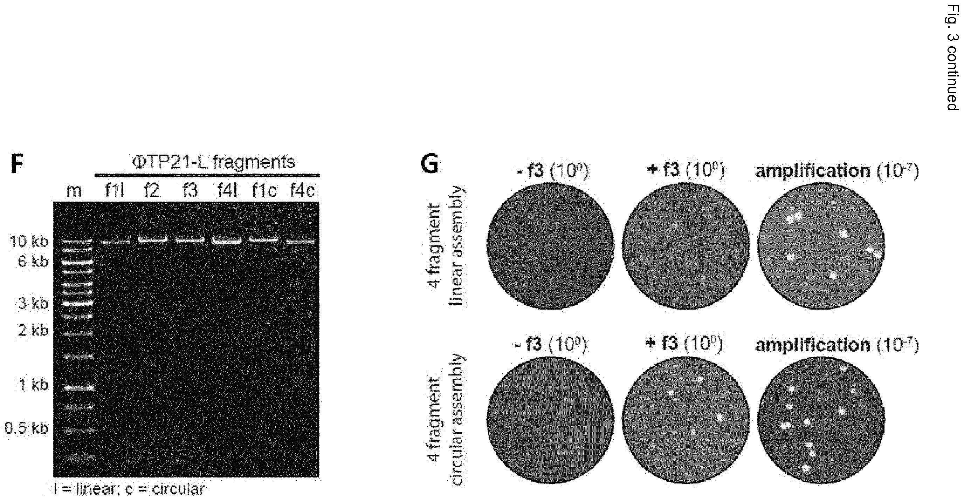

- Fig. 3: shows the rebooting of synthetic, *in vitro* assembled bacteriophage genomes in *Listeria* L-forms. The general workflow for rebooting of synthetic genomes in Rev2L is depicted in (A). Genomic phage DNA was extracted and purified from *Listeria monocytogenes* phage P35, *Listeria innocua* phage B025, and from *Bacillus cereus* phage TP21-L. Overlapping PCR-fragments covering the full genome were generated using a high-fidelity polymerase (B, D, F) and assembled *in-vitro* to generate circular molecules unless indicated otherwise. Assembly reactions were transformed and rebooted in Rev2L cells and plated on the respective indicator strains using incomplete assemblies as controls (C, E, G). For phage TP21-L, rebooting reactions were either assayed for phage production after 24 h using indicator strain or, where indicated (amplification), 10 µl of stationary HER1399 culture was added to the rebooting reaction at 6 h post transformation and assayed for phage production at 24 h (G).

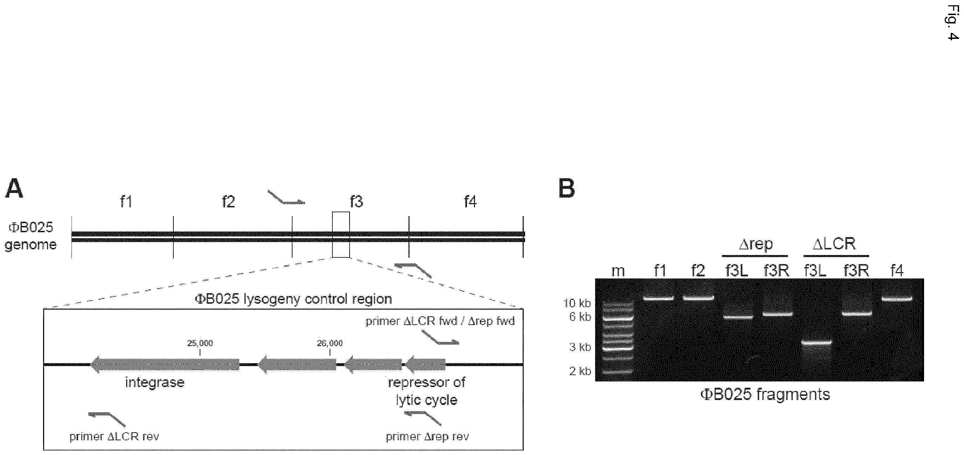

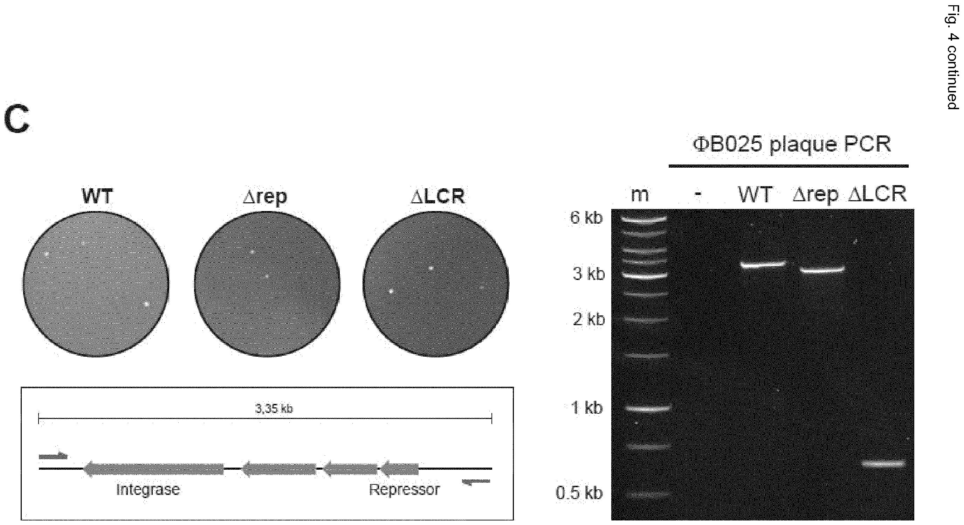

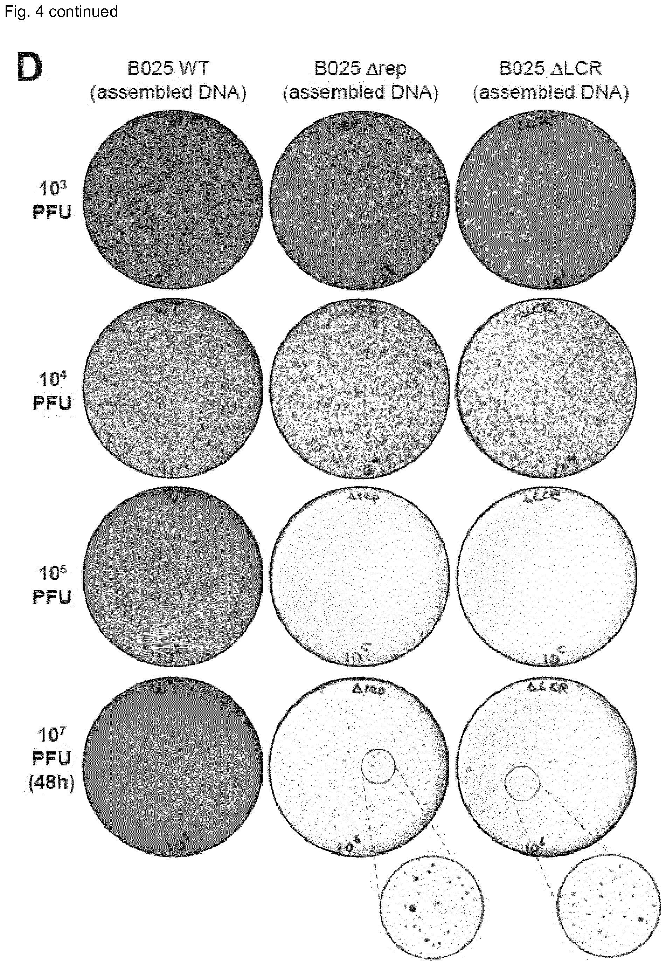

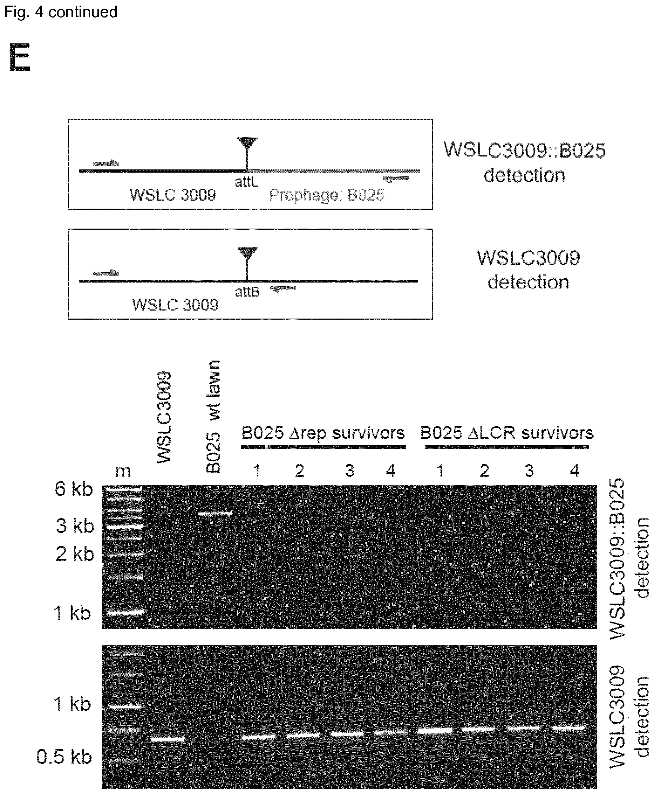

- Fig. 4: shows life-style conversion of temperate Listeria phage B025 from temperate to virulent. An *in-vitro* genome assembly strategy was used to produce mutants of the temperate *Listeria* phage B025 lacking either the repressor of integration (Δrep) or the complete lysogeny control region (ΔLCR). A workflow is shown in (A). Fragment three of the wild-type assembly contains the lysogeny control genes and was split into two overlapping fragments omitting either the repressor only or the complete LCR to yield five PCR fragments for genome assembly (B). Recombinant genomes were rebooted, the resulting phage mutants purified and assayed for correct genotype using PCR and sequencing (C). *L. ivanovii* WSLC3009 was infected with increasing numbers of wild-type, Δrep and ΔLCR phages using the soft-agar overlay technique. Surviving bacterial lawn (B025 wt) or single colonies (B025 Δrep and B025 ΔLCR) were assayed for the presence or absence of B025 prophage using the indicated primer pairs (E). LCR = lysogeny control region, PFU = plaque forming unit, attB = bacterial attachment site for prophage integration, attL = left prophage flanking region in WSLC3009:: B025.

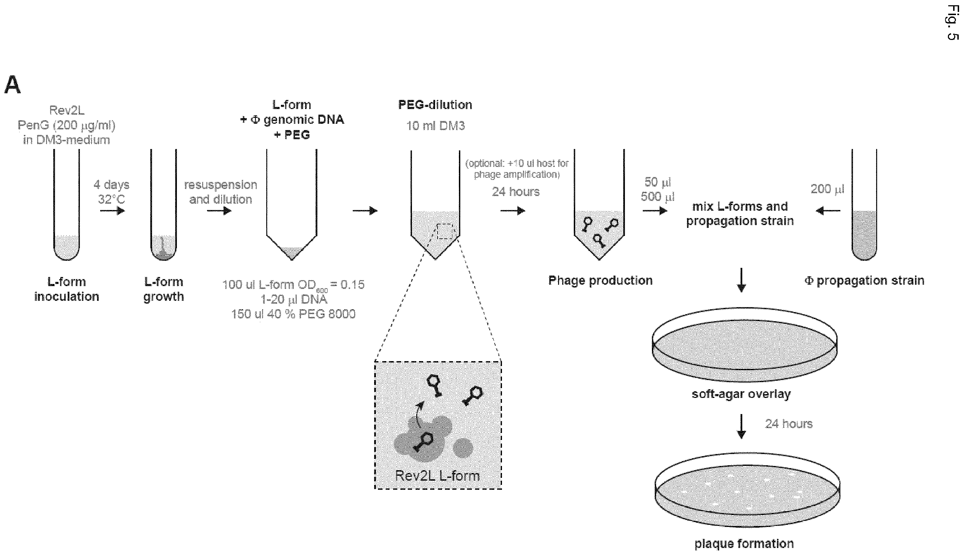

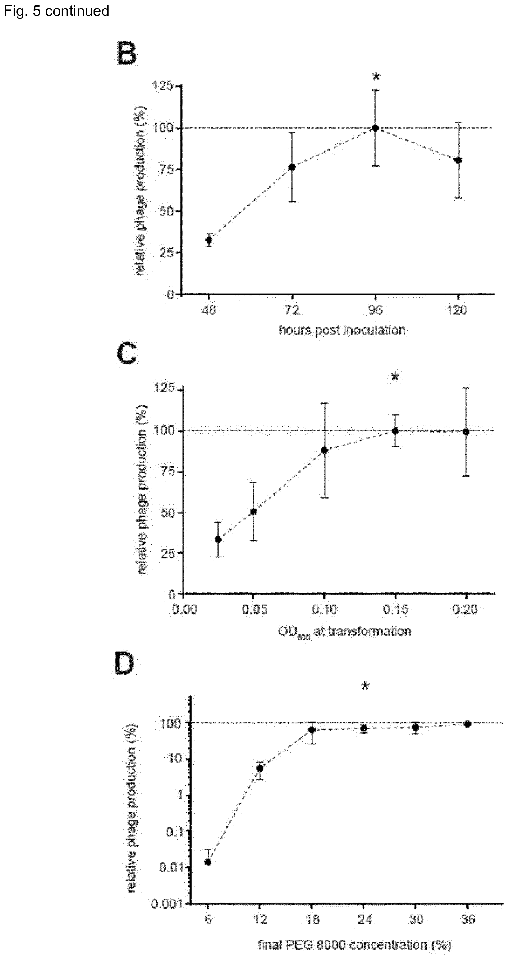

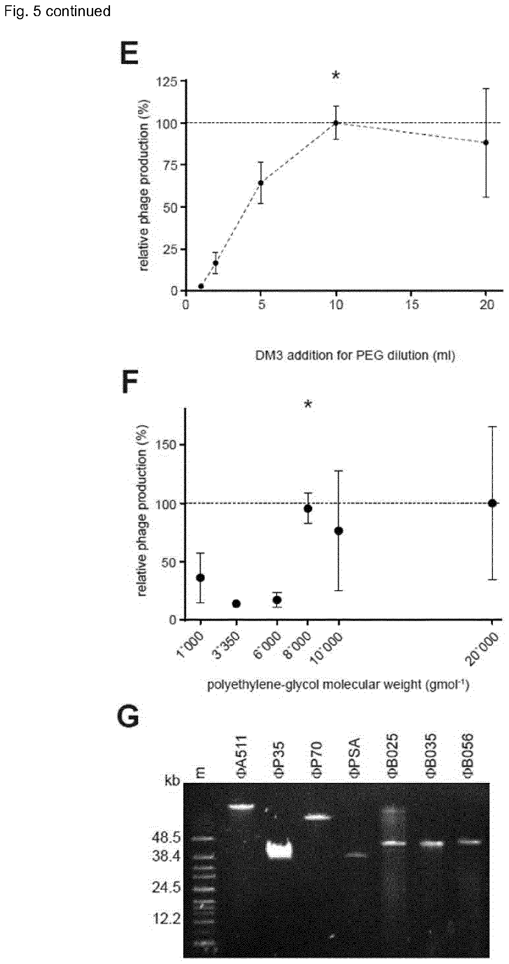

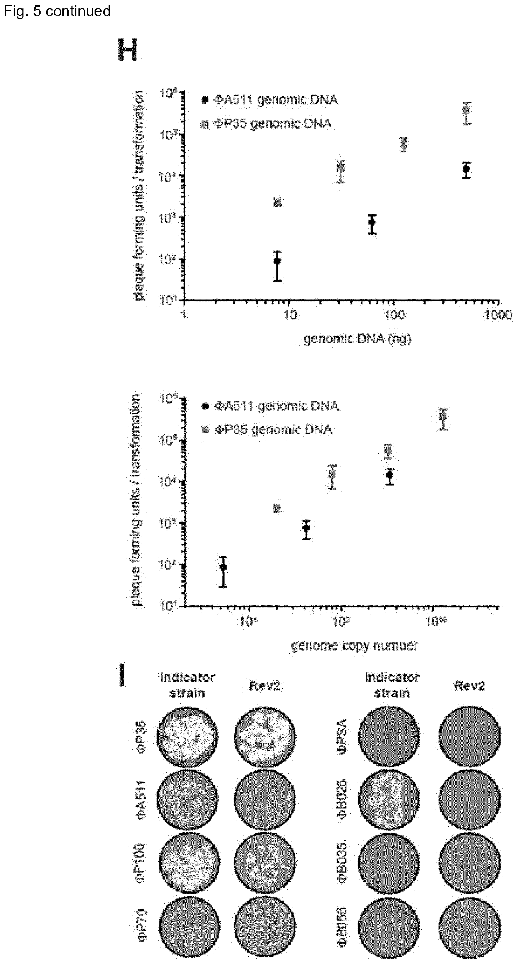

- Fig. 5: shows also the rebooting of *Listeria* bacteriophage genomes in L-form strain Rev2L. A detailed workflow for rebooting of phage genomes in which each parameter was optimized individually is shown in (A). Optimized parameters include: Time of Rev2L growth before transformation (B), adjusted optical density (600nm) of L-form culture at the time of transformation (C), final PEG concentration before addition of DM3 medium (D), volume of DM3 added to the DNA/L-form/PEG-mixture before incubation (E), and the average molecular weight of the PEG-chains used (F). The selected optimal conditions are indicated (asterisk). The quality of the bacteriophage DNA used for rebooting was assessed using pulsed-field gel electrophoresis (G). Using a dilution series of phage DNA, rebooting efficiency of phages P35 and A511 were compared (H). The ability of various *Listeria* phages to infect walled Rev2 cells was assessed by spotting phage dilutions on soft-agar overlays containing Rev2 or the phage indicator strain. (I) PenG = penicillin G, OD = optical density, PEG = polyethylene glycol. Data are mean ± SD (n=3).

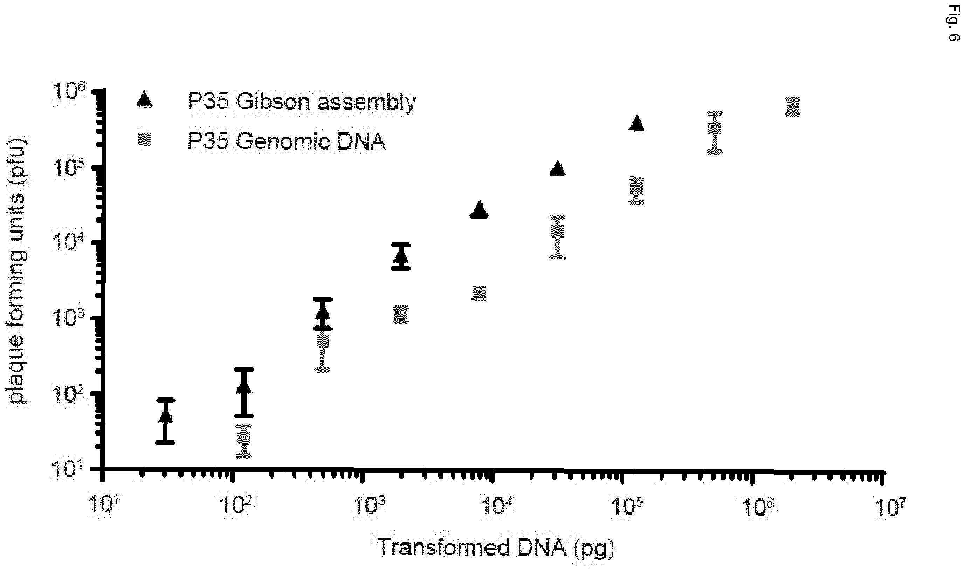

- Fig. 6: shows the rebooting of synthetic, *in vitro* assembled bacteriophage genomes in *Listeria* L-forms. The efficiency of genome rebooting using genomic DNA and *in vitro* assembled genomes as input material was compared: Four-fold dilution series of P35 gDNA or Gibson-assembly reaction were used for rebooting in Rev2L starting with 2 µg and 125 ng input material for gDNA and assembly reaction, respectively. Total pfu per transformation reaction were quantified and compared. Data is mean ± SD (n=3).

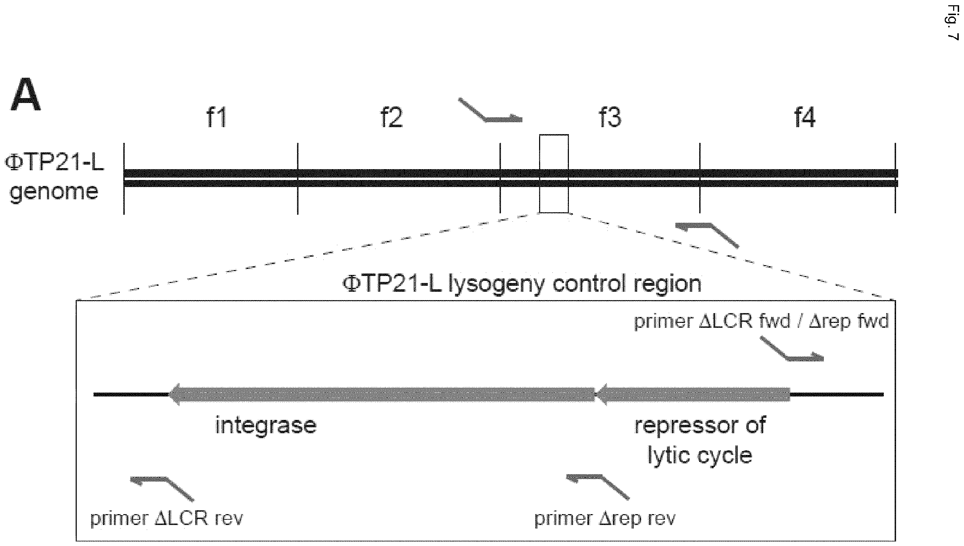

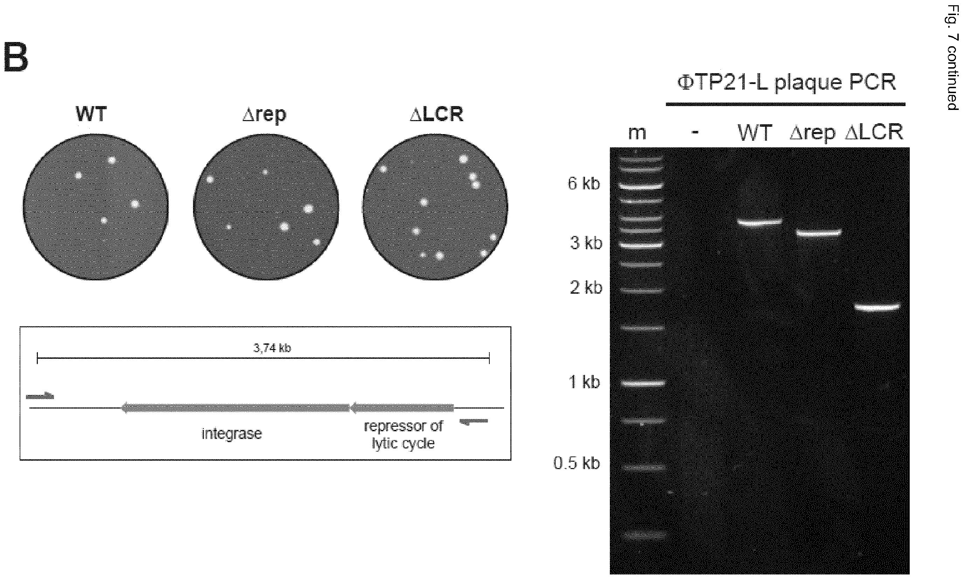

- Fig. 7: shows that deletion of TP21-L lysogeny control genes. An *in vitro* genome assembly strategy was used to produce mutants of the temperate *Bacillus* phage TP21-L lacking either the repressor of integration (Δrep) or the complete lysogeny control region (ΔLCR). A workflow is shown in (A). Fragment three of the wild-type assembly contains the lysogeny control genes and was split into two overlapping fragments omitting either the repressor only or the complete LCR to yield five PCR fragments for genome assembly. Recombinant genomes were rebooted, the resulting phage mutants purified and assayed for correct genotype using PCR and sequencing (B). LCR = lysogeny control region.

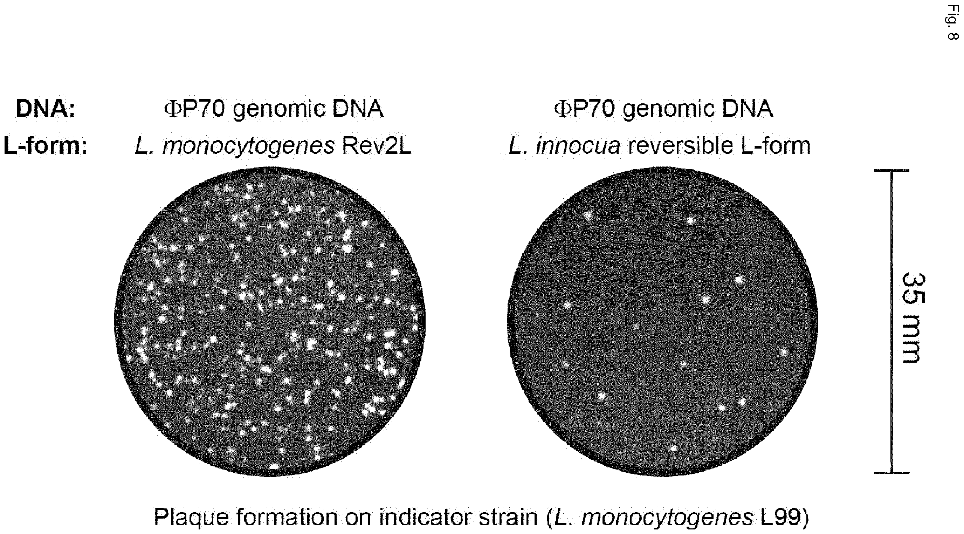

- Fig. 8: shows shows the rebooting of *Listeria* bacteriophage genomes in L-form strain Rev2 (*L. monocytogenes*) and in a reversible L-form that was obtained from non-pathogenic *Listeria innocua* 2021 (SLCC 5639). Each of the two L-form cultures was transformed with genomic DNA (1 µg) of the *L. monocytogenes* bacteriophage P70, incubated at 32 °C for 24 h, and tested for plaque formation using *L. monocytogenes* L99 as an indicator strain. Plaques on soft-agar overlays (24 h post infection) are shown.

### Examples

The present disclosure shows that a novel *Listeria monocytogenes* L-form strain Rev2L can be transformed with intact, purified bacteriophage DNA which leads to genome rebooting, i.e. the production of infectious virions from naked DNA. Rebooting was applicable to a heterogeneous group of *Listeria monocytogenes* and *Listeria innocua* phages independent of phage tropism, morphology, genome size or genome structure. Remarkably, this genome rebooting approach was also successful for several phages infecting *Bacillus* and *Staphylococcus,* effectively bypassing the genus barrier of infection. To generate both wild-type and recombinant viruses in a synthetic biology approach, bacteriophage genomes were assembled *in-vitro* using amplified overlapping fragments and subsequently rebooted in Rev2L cells. As proof of concept for this strategy, the inventors deleted lysogeny control genes of the temperate *Listeria* phage B025 and demonstrated an acquired virulent phenotype of the recombinant viruses. Combining *in-vitro* genome assembly and L-form transformation to engineer bacteriophages will facilitate the development of tailored phages both for basic and applied research on phages infecting Gram-positive pathogens.

Particularly, the below two-step protocol may be used for the generation of genetically-modified bacteriophages:
- In a first step, synthetic bacteriophage genomes are assembled *in-vitro* using a method based on Gibson-assembly, which is commercially available. Bacteriophage genomes are assembled as circular, double-stranded molecules mimicking DNA intermediates that occur during natural phage replication. In principle, any method that leads to the formation of a synthetic genome may be used for this first step and it is thus not limited to Gibson-assembled genomes.
- In a second step, *in-vitro* assembled viral genomes *l* synthetic genomes are transformed into *Listeria monocytogenes* cells where these synthetic phages are rebooted to produce infectious particles. For this transformation reaction, the inventors do not use wild-type *Listeria* cells but instead they relied on a newly identified *Listeria monocytogenes* L-form strain Rev2L. This strain has the ability to grow and divide in the absence of an intact cell-wall as long as an osmotically stabilized medium is used to prevent lysis of the bacterium. Due to the lack of an intact cell-wall, Rev2L can be transformed with large DNA-molecules such as intact bacteriophage genomes. For this transformation reaction, the inventors used an optimized, polyethylene glycol (PEG8000) based protocol. Transformed L-form bacteria were incubated for 24 hours to allow for phage rebooting. To isolate and amplify synthetic phages from these reactions, L-forms were subsequently mixed and incubated with the natural propagation strain of the phage of interest. It is not necessary to actively release progeny phage from the L-form cells because they are either lysed due to the activity of phage-encoded enzymes or simply by osmotic destabilization of the membrane.

Importantly, this method is not restricted to *Listeria* phages, but can be applied to other phages of Gram-positive bacteria or other bacteria.

### Detailed description of the proof of concept

To address phage genome rebooting in Gram-positive cells, the inventors used the virulent *Siphovirus* P35 that infects the food-borne pathogen *Listeria monocytogenes.* The 35'822 bp linear genomic DNA (gDNA) of P35 is too large for electroporation into walled *Listeria* cells which usually take up supercoiled plasmids of up to 10 kb at very low efficiencies. Therefore, the inventors were unable to address genome-rebooting using a conventional electroporation protocol with walled cells. Based on the assumption that the peptidoglycan envelope is the major barrier to transformation of larger DNAs, they investigated the possibility of using metabolically active, yet cell wall-deficient *Listeria* L-forms as recipients of purified, linear P35 gDNA. L-forms are induced by prolonged, repeated subcultivation in the presence of cell wall synthesis targeting antibiotics in an osmoprotective medium. For the method of the disclosure, the inventors used a novel *Listeria* strain Rev2 [Δ*lmo0584* Δ*lmo1653-54 Δlm01861*] that was obtained by long-term exposure of *L. monocytogenes* EGD-e to penicillinG (penG) in DM3 medium and has the ability to switch between growth as an L-form (designated Rev2L) and a walled bacterium (Rev2).

The inventors devised a work-flow for polyethylene-glycol (PEG)-mediated transformation of L-form bacteria with phage gDNA (Figure 1A and Figure 5A) and found that P35 is rebooted in a gDNA-, L-form-, and PEG-dependent process (Figure 1B). For this purpose, purified P35 gDNA was mixed with a growing penicillinG (penG)-induced Rev2L culture and PEG-8000 solution. PEG was subsequently diluted with osmotically stabilized DM3 medium and the mixture incubated for 24 h at 32 °C to allow for phage rebooting. The L-form transformation reaction was assayed for the presence of rebooted phage using the propagation strain of phage P35 as an indicator strain (*L*. *monocytogenes* Mack).

In order to get the highest phage yield, the inventors optimized each step of the rebooting protocol using P35 gDNA (see Figure 5 and Methods section for details). Under such optimized conditions, the inventors found a linear correlation between input DNA and phage production (Figure 1C) with a detection limit of 2.6 pg of P35 gDNA corresponding to about 66'000 genomes (assuming that the used DNA consisted of intact gDNA only). Besides P35, this L-form transformation protocol allowed for rebooting of a heterogeneous group of seven additional *Listeria* phages (Figure 1D). These include phages with virulent (P70, P100, and A511) and temperate (B025, B035, B056, PSA) life-styles, some phages with large genomes of more than 130 kb (P100, A511), and morphologically diverse phages with either contractile tails (Myoviruses; P100, A511) or non-contractile tails (Siphoviruses; all other). Phage B025 has cohesive overlapping genome ends (cos), whereas the other rebooted phages have terminally redundant genomes, with or without circular permutation.

These results strongly suggest that rebooting is independent of virion morphology, phage genome size and genome replication strategy. Moreover, the inventors were able to produce *Listeria* phages that would not normally infect a Rev2 cell (B025, B035, B056, and PSA, Figure 5) suggesting that receptor binding and/or genome translocation are the only barriers restricting the production of these phages during infection of walled cells. The diverse features of the rebooted *Listeria* phages are summarized in Figure 1D (small table) and show that rebooting in Rev2L is broadly applicable to *Listeria* phages.

The inventors compared the rebooting kinetics of phages P35, P70, and A511 (Figure 1E) and found that production of all three phages peaks at 24 h post transformation which is slow compared to infection kinetics in walled cells. For comparison, A511 has a burst time of 60 min when walled cells are infected. This large time difference is likely explained by slow metabolism in L-forms. Because L-forms are devoid of an intact cell wall, progeny virions are released by lysis of Rev2L cells either through the action of phage holin proteins or by osmotic destabilization when DM3 medium is diluted in osmotically non-stabilized soft-agar. This will also allow for the release of phages whose cell-wall lytic enzymes would not normally degrade *Listeria* serovar 1 cell walls. Released phages cannot bind to neighboring cells and their survival depends on stability in DM3 medium (Figure 1E).

In addition to rebooting of phages that infect *Listeria,* the inventors demonstrate that Rev2L cells can be used to reboot phages that infect different genera of Gram-positive organisms within the *Firmicutes* phylum. Phylogenetically, *Bacillus* is most closely related to *Listeria,* and the inventors found that the small, 37.46 kb Siphovirus TP21-L of *Bacillus cereus* as well as the large, 153.96 kb Myovirus Bastille of *Bacillus thuringiensis* could be rebooted in Rev2L using genomic DNA as a substrate (Figure 2A).

Next, the inventors found that *Listeria* L-forms could also be used as a rebooting platform for phages infecting the human pathogen *Staphylococcus aureus:* They successfully rebooted 2638A, a 41.32 kb Siphovirus as well as phage K, a large 127.40 kb Myovirus (Figure 2B). The results so far demonstrate that Rev2L is a highly versatile host for the uptake of large viral genomes and the cross-genus rebooting of phages of Gram-positive organism from naked, linear gDNA.

Next, the inventors utilized the L-form platform of the disclosure to address whether synthetic genomes could also be used as a substrate for rebooting (workflow is shown in Figure 3A). First, they amplified and purified overlapping segments of P35 DNA and assembled synthetic genomes using the Gibson method. Because most phages use circular replication-intermediates, the overlaps between fragments were designed to allow for end-joining (circular closure). Nevertheless, the formation of concatemeric or terminally redundant linear DNA cannot be excluded. To assemble the P35 genome, the inventors used either six fragments of about 6 kb or three fragments of about 12 kb (Figure 3B) and it was found that P35 was efficiently rebooted from synthetic DNA (Figure 3C). As control, they used an incomplete assembly lacking one fragment. For P35, using synthetic DNA was even more efficient compared to purified gDNA (detection limit = 1.1 pg DNA, SFigure 6), either because ring-closure is no longer required or because circular DNA is transformed more efficiently than a linear molecule.

Besides P35, the inventors also used this synthetic approach to assemble and reboot the genomes of the temperate *L. innocua* phage B025 (Figure 3D-E). *In-vitro* assembly and cross-genus rebooting of *B. cereus* phage TP21-L (Figure 3F-G) was also successful in Rev2L and it was found that the production of phage was amplified when host cells were added to the rebooting reaction at 6 h post transformation (Figure 3G). This amplification strategy was not successful for any phages infecting *Listeria* or *Staphylococcus* because they fail to infect in DM3 medium. Also TP21-L was used to show that synthetic phages are produced even in the absence of a designed ring-closure (Figure 3G).

The unique combination of synthetic genome assembly and rebooting in L-forms offers a platform for phage genome engineering: To explore this approach, the inventors attempted to switch the life style of a temperate *Listeria* phage from lysogenic to lytic using a synthetic, modified genome. To this end, they assembled the genome of temperate *Listeria* phage B025 but omitted genes that control and mediate prophage integration. In B025, these genes are encoded on a 2.7 kb putative lysogeny control region (LCR), and either the repressor of the lytic cycle (B025 Δrep) or the whole LCR (B025 ΔLCR) including the phage integrase were deleted. The genome assembly strategy is depicted in Figure 4A and fragments for the assembly of phage mutants are shown in Figure 4B. Recombinant phage genomes were assembled, rebooted successfully, and the resulting phages were validated by PCR and sequencing of the LCR region (Figure 4C). Although there were no obvious differences in plaque morphology (Figure 4C), the recombinants could no longer integrate and replicated as synthetic "virulent" phages (Figure 4D): When rebooted phage from a B025 wild-type assembly was used, prophage integration effectively prevented killing of the host at high multiplicities of infection (Figure 4D; 10⁵ pfu). As a result, a seemingly uninfected bacterial lawn was observed which consisted of B025 lysogens (WSLC3009::B025) that were resistant to super-infection. In contrast, the B025 mutants displayed enhanced killing and were able to lyse almost all host cells in a soft-agar overlay assay at identical multiplicity of infection. Only a few survivors grew after infection with lysogeny-control mutants. When regrown, these survivors were not resistant to B025 and had an empty integration site (Figure 4E) proofing that the LCR mutants were indeed lytic and unable to integrate into the host genome. A similar approach was successfully applied to *B. cereus* phage TP21-L, where the complete 2 kb LCR or the repressor only were deleted (Figure 7). Switching phage life-style from temperate to virulent is a fast, broadly applicable method to increase the arsenal of lytic phages with enhanced antimicrobial activity that could potentially be used in a biomedical setting or for pathogen detection.

The genome engineering platform of the disclosure allows for a rational design and fast, reporter-free production of recombinant bacteriophages. In contrast to recombination-based technologies, cumbersome screening for recombinant phages is no longer required. In the future, this technology will enable us to tailor phages with enhanced antimicrobial properties, incorporate sensitive reporter genes into phage genomes for pathogen detection, and possibly allow for host-range modifications by switching receptor binding proteins. Many aspects of basic phage biology are still poorly understood, mostly due to the lack of efficient genetic tools for mutation, deletion, and molecular tagging of phage proteins. This is particularly true for virulent phages of Gram-positive bacteria. Therefore, the approach presented here will contribute substantially to an enhanced understanding of the biology of these nano-machines and pave the way for novel phage-based biotechnological applications beyond their use as biocontrol- and detection-agents.

### Materials and methods

### Bacterial strains and growth conditions

*Listeria monocytogenes* WSLC1042 and Mack, *Listeria ivanovii* WSLC3009, *Bacillus thruingiensis* HER1211, and *Bacillus cereus* HER1399 were grown at 30 °C in 0.5x BHI medium. *Staphylococcus aureus* ATCC19685 and *Staphylococcus aureus* 2638A were grown at 37°C in 0.5x BHI medium. Novel *Listeria monocytogenes* L-form strain Rev2L was grown at 32°C in a slightly modified version of DM3 medium for which we used tryptone instead of casamino acids (5 gL⁻¹ tryptone, 5 gL⁻¹ yeast extract, 0.01 % BSA, 500 mM succinic acid, 5 gL⁻¹ glucose, 20 mM K₂HPO₄, 11 mM KH₂PO₄, 20 mM MgCl2 adjusted to pH 7.3).

### Bacteriophage propagation and DNA extraction

Phages were propagated using the soft-agar overlay method and extracted from plates with SM buffer (100 mM NaCl, 8 mM MgSO₄, and 50 mM Tris pH 7.4). Phage P35 was propagated in *L. monocytogenes* Mack at room-temperature using LC (LB agar supplemented with 10 mM CaCl₂) as bottom and top agar (LC/LC). All other phages were propagated using 0.5x BHI as the bottom- and LC as the top-agar. Phage PSA was propagated in *L. monocytogenes* WSLC1042 at 30 °C and all other *Listeria* phages were propagated on *L. ivanovii* WSLC3009 at 30°C. *Bacillus* phages Bastille and TP21-L were propagated at 30°C on HER1211 and HER1399, respectively while *Staphylococcus aureus* phages K and 2638A were propagated at 37 °C on ATCC19685 and S2638A, respectively. For DNA extraction, filter-sterilized lysates were digested with DNasel (10 µgml⁻¹) and RNaseA (1 U in 10 ml) for 30 min at 37 °C. Phages were subsequently concentrated by PEG precipitation (7% PEG8000 and 1 M NaCl), digested with proteinase K (200 ug/ml, 50°C, 30 min, in SM buffer + 10 mM EDTA pH 8), and purified using the High Pure Viral Nucleic Acid Kit (Roche Life Science). To purify DNA from large bacteriophage genomes (ΦP100, ΦA511, ΦBastille, ΦK), PEG-precipitated phage was purified using stepped CsCI gradient ultracentrifugation, dialyzed against a 1000x excess of SM buffer, digested with proteinase K, and DNA was extracted using organic solvents as previously described.

### Generation of Rev2 strain and reversible L. innocua L-Form strains

Briefly, an overnight culture of *Listeria* EGD-e or *Listeria innocua* 2021 (SLCC 5639) was plated on DM3 agar supplemented with penicillin (200 µg*ml⁻¹). After approximately two weeks of incubation, single colonies emerged which were transferred to liquid DM3 medium containing penicillin G (PenG) and further incubated without shaking. It took around one week until the bacteria grew in liquid medium for the first time. After growth of L-forms in liquid DM3+PenG medium, they were passaged into liquid DM3 medium without penicillin G. The resulting L-forms did not revert to their walled form in DM3 liquid medium, but only on DM3 agar plates (in absence of penicillin G). After two days of growth in liquid DM3 medium, the L-forms were plated on DM3 plates without penicillin, where revertant colonies (walled cells) usually emerged after 2-5 days. These were picked and inoculated into liquid BHI medium, in which any residual L-form cells would burst due to the absence of osmotic protectants. From this culture, a cryo stock was be prepared and the strain tested for its ability to grow in DM3 medium supplemented with penicillin G. The reversible L-form generated with EGD-e is referred to as Rev2 (walled) or Rev2L (L-form).

### L-form transformation and genome rebooting

Induction of Rev2 cells into the L-form state was performed in the presence of 200 µg*ml⁻¹ PenG in DM3 medium at 32 °C, and L-form cells were passaged every 3-4 days by 1:1000 dilution in fresh DM3 medium. The different L-form passages were assayed for their ability to produce infective phage particles upon transformation. We observed that phage production was maximal for passage five and therefore used a cryo stock (-80°C) prepared from this passage for all experiments. Small volumes (4-5 ul) of the cryo-culture were inoculated into 1 ml prewarmed DM3 + PenG, suspended using a vortex mixer at low speed, and incubated without agitation at 32 °C for 96 h. The resulting L-form culture was suspended by pipetting and adjusted to an OD₆₀₀ₙₘ of 0.15 using DM3 medium. 100 µl OD-adjusted Rev2L culture was mixed with 10-20 µl phage genomic DNA in a 50 ml Falcon tube. 150 ul sterile 40% PEG8000 solution was added and mixed thoroughly by pipetting. After 5 min of incubation, 10 ml pre-warmed DM3 medium was added, suspended using a serological pipette, and the transformation reaction was incubated without agitation at 32 °C for 24 h unless indicated otherwise. The L-form transformation reaction was re-suspended by pipetting, and assayed for matured phage particles using the soft-agar overlay method: 5 ml of molten LC soft-agar were mixed with 50 - 500 µl of the transformation reaction and 200 µl of a fresh stationary-phase culture of a suitable phage propagation strain (the indicator strain). This soft-agar mixture was poured onto solid agar plates and incubated at the optimal growth temperature of the indicator strain to allow for phage propagation and visible plaque formation.

### In-vitro assembly of synthetic genomes

Bacteriophage genomes were divided *in silico* into three to six fragments of similar size carrying 40 bp overlapping ends. Fragments were chosen randomly for phage genomes with circular permutation. For phages with non-permuted genomes (TP21-L and B025), genome fragments were designed to allow for artificial circularization at their physical ends using long overlapping primers. Genome fragments were amplified from phage gDNA by PCR using Phusion DNA polymerase (Thermo Scientific) and subsequently purified using silica columns. Synthetic genomes were assembled for 1 h at 50 °C using the NEBuilder HiFi DNA Assembly Cloning Kit (NewEngland Biolabs). For every assembly reaction, 150 - 250 ng purified DNA was used per fragment in a 20 µl reaction and 15 µl were used for rebooting.

## Claims

1. Method for producing or propagating an engineered bacteriophage, comprising the steps of:
- providing a functional synthetic genome of an engineered bacteriophage being able to infect a target bacterium,
- providing a recipient bacterium,
- transforming said recipient bacterium with said functional synthetic genome in a transformation step, yielding a transformed recipient bacterium,
- incubating said transformed recipient bacterium in a first incubation step, wherein said engineered bacteriophage is propagated within said transformed recipient bacterium, and
- further incubating said transformed recipient bacterium or said propagated engineered bacteriophage released from said transformed recipient bacterium with a target bacterium in a second incubation step, wherein said propagated engineered bacteriophage infects said target bacterium and is further propagated within said target bacterium,
wherein said recipient bacterium is a cell wall-deficient bacterium.

2. The method according to claim 1, wherein said recipient bacterium is a cell wall-deficient variant of or derived from a Gram-positive bacterium, particularly a cell wall-deficient variant of or derived from a bacterium of the genus *Listeria,* particularly *Listeria monocytogenes.*

3. The method according to any one of the preceding claims, wherein said target bacterium is a Gram-positive bacterium, particularly selected from the genus of *Listeria, Bacillus, Enterococcus, Streptococcus, Clostridium* or *Staphylococcus,* more particular *Listeria monocytogenes, Listeria ivanovii, Listeria innocua, Bacillus subtilis, Bacillus cereus, Bacillus thuringiensis* or *Staphylococcus aureus.*

4. The method according to any one of the preceding claims, wherein said recipient bacterium and said target bacterium belong to or are derived from different species or members of different genera.

5. The method according to any one of the preceding claims, wherein said functional synthetic genome is provided by *in vitro* or *in vivo* assembly of fragments thereof or by *de novo* synthesis.

6. The method according to any one of the preceding claims, wherein said functional synthetic genome has a length of at least 10,000 base pair, particularly at least 30,000 base pairs, more particularly at least 40,000 base pair.

7. The method according to any one of the preceding claims, wherein said transformation step is conducted in presence of a polyethylene glycol, particular with a mean molecular weight in the range of 1,000 g*mol⁻¹ to 30,000 g*mol⁻¹, more particular in the range of 7,000 g*mol⁻¹ to 20,000 g*mol⁻¹, particularly PEG-8000.

8. The method according to any one of the preceding claims, wherein said first incubation step is conducted over a period in the range of 4 h to 96 h, particularly in the range of 24 h to 32 h.

9. The method according to any one of the preceding claims, wherein said recipient bacterium is provided by incubating a cell walled precursor bacterium in presence of a cell wall synthesis interfering antibiotic, particularly selected from beta lactam antibiotics, glycopeptide antibiotics, cycloserine, or fosfomycin, and optionally a osmoprotective medium, yielding said recipient bacterium.

## Patentansprüche

1. Verfahren zum Herstellen oder Propagieren eines manipulierten Bakteriophagen, das die folgenden Schritte umfasst:
- Bereitstellen eines funktionellen synthetischen Genoms eines manipulierten Bakteriophagen, der in der Lage ist, ein Zielbakterium zu infizieren,
- Bereitstellen eines Empfängerbakteriums,
- Transformieren des besagten Empfängerbakteriums mit dem besagten funktionellen synthetischen Genom in einem Transformationsschritt, wodurch ein transformiertes Empfängerbakterium entsteht,
- Inkubieren des besagten transformierten Empfängerbakteriums in einem ersten Inkubationsschritt, wobei der besagte manipulierte Bakteriophage innerhalb des besagten transformierten Empfängerbakteriums propagiert wird, und
- weiteres Inkubieren des besagten transformierten Empfängerbakteriums oder des besagten propagierten manipulierten Bakteriophagen, der aus dem besagten transformierten Empfängerbakterium freigesetzt wurde, mit einem Zielbakterium in einem zweiten Inkubationsschritt, wobei der besagte propagierte manipulierte Bakteriophage das besagte Zielbakterium infiziert und innerhalb des besagten Zielbakteriums weiter propagiert wird,
wobei das besagte Empfängerbakterium ein Zellwand-defizientes Bakterium ist.

2. Verfahren nach Anspruch 1, wobei das besagte Empfängerbakterium eine Zellwand-defiziente Variante eines gram positiven Bakteriums oder von diesem abgeleitet ist, insbesondere eine Zellwand-defiziente Variante eines Bakteriums der Gattung Listeria, insbesondere Listeria monocytogenes, oder von diesem abgeleitet.

3. Verfahren nach einem der vorhergehenden Ansprüche, wobei das besagte Zielbakterium ein grampositives Bakterium ist, besonders ausgewählt aus der Gattung *Listeria, Bacillus, Enterococcus, Streptococcus, Clostridium* oder *Staphylococcus,* insbesondere *Listeria monocytogenes, Listeria ivanovii, Listeria innocua, Bacillus subtilis, Bacillus cereus, Bacillus thuringiensis* oder *Staphylococcus aureus.*

4. Verfahren nach einem der vorhergehenden Ansprüche, wobei das besagte Empfängerbakterium und das besagte Zielbakterium zu verschiedenen Arten oder Mitgliedern verschiedener Gattungen gehören oder von diesen abgeleitet sind.

5. Verfahren nach einem der vorhergehenden Ansprüche, wobei das besagte funktionelle synthetische Genom durch *In-vitro-* oder *In-vivo*-Assemblierung von Fragmenten davon oder durch *De-novo*-Synthese bereitgestellt wird.

6. Verfahren nach einem der vorhergehenden Ansprüche, wobei das besagte funktionelle synthetische Genom eine Länge von mindestens 10.000 Basenpaaren, besonders mindestens 30.000 Basenpaaren, insbesondere mindestens 40.000 Basenpaaren aufweist.

7. Verfahren nach einem der vorhergehenden Ansprüche, wobei der besagte Transformationsschritt in Gegenwart eines Polyethylenglykols ausgeführt wird, besonders mit einem mittleren Molekulargewicht im Bereich von 1.000 g*mol⁻¹ bis 30.000 g*mol⁻¹, insbesondere im Bereich von 7.000 g*mol⁻¹ bis 20.000 g*mol⁻¹, insbesondere PEG-8000.

8. Verfahren nach einem der vorhergehenden Ansprüche, wobei der besagte erste Inkubationsschritt über einen Zeitraum im Bereich von 4 h bis 96 h, besonders im Bereich von 24 h bis 32 h durchgeführt wird.

9. Verfahren nach einem der vorhergehenden Ansprüche, wobei das besagte Empfängerbakterium durch Inkubieren eines Zellwandvorläuferbakteriums in Gegenwart eines die Zellenwandsynthese störenden Antibiotikums bereitgestellt wird, besonders ausgewählt aus Beta-Lactam-Antibiotika, Glycopeptid-Antibiotika, Cycloserin oder Fosfomycin und optional einem osmoprotektiven Medium, wodurch das besagte Empfängerbakterium entsteht.

## Revendications

1. Procédé de production ou de propagation d'un bactériophage modifié, comprenant les étapes de :
- fournir un génome synthétique fonctionnel d'un bactériophage modifié étant capable d'infecter une bactérie cible,
- fournir une bactérie réceptrice,
- transformer ladite bactérie réceptrice avec ledit génome synthétique fonctionnel dans une étape de transformation, donnant une bactérie réceptrice transformée,
- incuber ladite bactérie réceptrice transformée dans une première étape d'incubation, où ledit bactériophage modifié est propagé à l'intérieur de ladite bactérie réceptrice transformée, et
- incuber en outre ladite bactérie réceptrice transformée ou ledit bactériophage modifié propagé libéré de ladite bactérie réceptrice transformée avec une bactérie cible dans une deuxième étape d'incubation, où ledit bactériophage modifié propagé infecte ladite bactérie cible et est en outre propagé à l'intérieur de ladite bactérie cible,
où ladite bactérie réceptrice est une bactérie à paroi cellulaire déficiente.

2. Procédé selon la revendication 1, où ladite bactérie réceptrice est une variante à paroi cellulaire déficiente d'une bactérie Gram positive ou dérivée d'une bactérie Gram positive, en particulier une variante à paroi cellulaire déficiente d'une bactérie du genre *Listeria* ou dérivée d'une bactérie du genre *Listeria,* en particulier *Listeria monocytogenes.*

3. Procédé selon l'une quelconque des revendications précédentes, où ladite bactérie cible est une bactérie Gram positive, en particulier sélectionnée du genre *Listeria, Bacillus, Enterococcus, Streptococcus, Clostridium* ou *Staphylococcus,* plus particulièrement *Listeria monocytogenes, Listeria ivanovii, Listeria innocua, Bacillus subtilis, Bacillus cereus, Bacillus thuringiensis* ou *Staphylococcus aureus.*

4. Procédé selon l'une quelconque des revendications précédentes, où ladite bactérie réceptrice et ladite bactérie cible appartiennent à ou sont dérivées d'espèces différentes ou de membres de genres différents.

5. Procédé selon l'une quelconque des revendications précédentes, où ledit génome synthétique fonctionnel est fourni par un assemblage *in vitro* ou *in vivo* de fragments de celui-ci ou par une synthèse *de novo.*

6. Procédé selon l'une quelconque des revendications précédentes, où ledit génome synthétique fonctionnel a une longueur d'au moins 10 000 paires de bases, en particulier d'au moins 30 000 paires de bases, plus particulièrement d'au moins 40 000 paires de bases.

7. Procédé selon l'une quelconque des revendications précédentes, où ladite étape de transformation est conduite en présence d'un polyéthylène glycol, en particulier avec un poids moléculaire moyen compris entre 1 000 g*mol⁻¹ et 30 000 g*mol⁻¹, plus particulièrement compris entre 7 000 g*mol⁻¹ et 20 000 g*mol⁻¹ en particulier PEG-8000.

8. Procédé selon l'une quelconque des revendications précédentes, où ladite première étape d'incubation est conduite sur une période comprise entre 4 h et 96 h, en particulier comprise entre 24 h et 32 h.

9. Procédé selon l'une quelconque des revendications précédentes, où ladite bactérie réceptrice est fournie en incubant une bactérie précurseur à paroi cellulaire en présence d'un antibiotique interférant avec la synthèse de la paroi cellulaire, en particulier sélectionné parmi antibiotiques bêta-lactamines, antibiotiques glycopeptides, cyclosérine, ou fosfomycine, et facultativement un milieu osmoprotecteur, donnant ladite bactérie réceptrice.
